Europäisches Patentamt

European Patent Office

Office européen des brevets

(11) Veröffentlichungsnummer: **0 008 654**
**A1**

# EUROPÄISCHE PATENTANMELDUNG

(21) Anmeldenummer: 79102589.3

(22) Anmeldetag: 23.07.79

(51) Int. Cl.³: **C 07 C 29/82**
**C 07. C 31/40**

(30) Priorität: 25.07.78 DE 2832532

(43) Veröffentlichungstag der Anmeldung:
19.03.80 Patentblatt 80/6

(84) Benannte Vertragsstaaten:
BE DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: König, Inge
Luitpoldallee 13
D-8260 Mühldorf(DE)

(72) Erfinder: Streitberger, Horst
Josef-Hofmiller-Weg 9
D-8262 Altötting(DE)

(72) Erfinder: Krempl, Engelbert
Hochgernweg 2
D-8261 Burgkirchen/Alz(DE)

(72) Erfinder: Schwenk, Ulrich, Dr.
Robert-Koch-Strasse 209
D-8263 Burghausen/Salzach(DE)

(54) Verfahren zur Gewinnung von reinen 2-(Perfluoralkyl)-äthanolen aus ihren Gemischen mit 2-(Perfluoralkyl)-äthylenen und gegebenenfalls 2-(Perfluoralkyl)-äthylestern.

(57) 2-(Perfluoralkyl)-äthanole der Formel $R_fCH_2CH_2OH$ werden aus 2-(Perfluoralkyl)-äthyljodiden der Formel $R_fCH_2CH_2J$ gewonnen, wobei sie im Gemisch mit 2-(Perfluoralkyl)-äthylenen der Formel $R_fCH=CH_2$ und gegebenenfalls auch mit 2-(Perfluoralkyl)-äthylcarbonsäureestern der Formel $R_fCH_2CH_2OCOR$ entstehen. Aus diesen Gemischen können die 2-(Perfluoralkyl)-äthanole in hoher Reinheit gewonnen werden, wenn bei der destillativen Trennung als Schleppmittel ein Alkanol mit 1 bis 8 C-Atomen oder ein Monoalkyläther des Äthylenglykols mit einem Alkylrest von 1 bis 4 C-Atomen oder ein Gemisch solcher Alkanole und/oder Alkylglykole zugesetzt wird, bei Anwesenheit von 2-(Perfluoralkyl)-äthylcarbonsäureestern ferner ein saurer Umesterungskatalysator.

EP 0 008 654 A1

- 1 -

HOECHST AKTIENGESELLSCHAFT    HOE 78/F 917        Dr.SC/ei

Verfahren zur Gewinnung von reinen 2-(Perfluoralkyl)-
äthanolen aus ihren Gemischen mit 2-(Perfluoralkyl)-
äthylenen und gegebenenfalls 2-(Perfluoralkyl)-äthyl-
estern

Die Erfindung betrifft ein Verfahren zur Gewinnung von
2-(Perfluoralkyl)-äthanolen der Formel $R_f-CH_2CH_2OH$ (im
folgenden kurz als $R_f$-äthanole bezeichnet) aus deren
Gemischen mit 2-(Perfluoralkyl)-äthylenen der Formel
$R_f-CH=CH_2$ (im folgenden kurz als $R_f$-äthylene bezeichnet)
und gegebenenfalls mit 2-(Perfluoralkyl)-äthylcarbonsäure-
estern der Formel $R_f-CH_2CH_2OCOR$ (im folgenden kurz als
$R_f$-äthylester bezeichnet), wobei $R_f$ ein Gemisch aus Perfluoralkylresten mit 4 bis 24, vorzugsweise 4 bis 16 C-
Atomen entweder endständig methylverzweigt oder vorzugsweise geradkettig, und wobei R Wasserstoff, einen aliphatischen oder aromatischen Rest bedeutet.

Ausgangsprodukte für die Gewinnung von $R_f$-äthanolen sind
die 2-(Perfluoralkyl)-äthyljodide der Formel $R_f-CH_2CH_2J$,
die wiederum aus Perfluoralkyljodiden der Formel $R_fJ$ durch
Anlagerung von Äthylen gewonnen werden. Die Perfluoralkyljodide ihrerseits entstehen durch Telomerisationsreaktion eines kurzkettigen Perfluoralkyljodids (Perfluormethyl-, -äthyl-, -propyl- oder -isopropyljodid) mit Tetrafluor-äthylen. Aus praktischen Gründen wird aus dem entstehenden Telomerisationsgemisch üblicherweise nur das Ausgangsprodukt und gegebenenfalls der Telomerisatanteil mit $R_f$-Resten bis zu 3 C-Atomen abgetrennt, während das restliche Telomerisationsgemisch entweder insgesamt oder aber zerlegt in größere Fraktionen (also mit breiten Kettenverteilungen hinsichtlich des $R_f$-Rests) der Weiterverarbeitung (Anlagerung von Äthylen und Herstellung von Folgeprodukten) unterworfen wird.

Für die Herstellung von $R_f$-äthanolen aus $R_f$-äthyljodiden sind eine Reihe von Verfahren bekannt, bei denen als Nebenprodukte $R_f$-äthylene erhalten werden, beispielsweise aus den deutschen Offenlegungsschriften 23 18 677 und 23 18 941. Obwohl sich die Bildung dieser $R_f$-äthylene zwar weitgehend, jedoch nie völlig unterdrücken läßt, sind solche Verfahren dennoch von großem Interesse, da die $R_f$-äthylene ihrerseits weiterverarbeitet werden können. Voraussetzung ist jedoch, daß es gelingt, die beiden Komponenten $R_f$-äthanole und $R_f$-äthylene in möglichst reiner Form zu trennen.

Als Nebenprodukte, teilweise sogar in sehr hohen Anteilen, fallen nach den Verfahren der DE-PS 1 264 444 und der DE-OS 23 18 941 ferner $R_f$-äthylester an, die durch Veresterung des $R_f$-äthanols mit dem als Reaktanten anwesenden Carbonsäurederivat gebildet werden. Dies können Carbonsäurederivate von Carbonsäuren der verschiedensten Art sein (vgl. DE-PS 1 264 444, Spalte 6, Zeilen 38 bis 56), bevorzugt sind jedoch Derivate von kurzkettigen Alkancarbonsäuren mit einer Alkylkette von 1 bis 4 C-Atomen. Da diese $R_f$-äthylester die Ausbeuten der gewünschten $R_f$-äthanole ver-mindern, ist es anzustreben, sie in $R_f$-äthanole zurückzu-verwandeln.

Die Trennung der erhaltenen Gemische von $R_f$-äthanolen und $R_f$-äthylenen gelingt – beispielsweise durch Waschen mit Wasser zur Abtrennung wasserlöslicher Bestandteile und anschließende Abtrennung der $R_f$-äthylene durch fraktionierte Destillation - dann in befriedigender Weise, wenn es sich um $R_f$-kettenreine Produkte handelt, d. h. die beiden Komponenten einen einzelnen und den gleichen $R_f$-Rest enthalten.

Werden jedoch, wie oben erläutert, die üblicherweise eingesetzten Telomerisationsprodukte mit $R_f$-Kettenschnitten zugrundegelegt, so wird eine destillative Trennung praktisch unmöglich, wie beispielsweise der Vergleich folgender Siedepunkte zeigt:

| $R_f$-Rest | $R_f$-äthanol Kp/12 mbar | $R_f$-äthylen Kp/12 mbar |
|---|---|---|
| $C_6F_{13}-$ | 72 - 77 °C | 20 - 25 °C |
| $C_8F_{17}-$ | 93 - 98 °C | 35 - 40 °C |
| $C_{10}F_{21}-$ | 112 - 120 °C | 65 - 75 °C |
| $C_{12}F_{25}-$ | 127 - 137 °C | 95 - 98 °C |

Wie ersichtlich, siedet beispielsweise $C_6F_{13}CH_2CH_2OH$ zusammen mit $C_{10}F_{21}CH=CH_2$ und ebenso $C_8F_{17}CH_2CH_2OH$ zusammen mit $C_{12}F_{25}CH=CH_2$ im jeweils etwa gleichen Temperaturintervall.

Es bestand daher die Aufgabe, ein Verfahren zu finden, mit dem die Trennung solcher Gemische gelingt und gleichzeitig die gegebenenfalls anwesenden $R_f$-äthylester in $R_f$-äthanole umgewandelt werden können.

Die Aufgabe wird erfindungsgemäß gelöst durch ein Verfahren, das dadurch gekennzeichnet ist, daß man den Gemischen ein Alkanol mit 1 bis 8 C-Atomen oder einen Monoalkyläther des Äthylenglykols, dessen Alkylrest 1 bis 4 C-Atome besitzt, als Schleppmittel zusetzt, und bei Anwesenheit von 2-(Perfluoralkyl)-äthylcarbonsäureestern ferner einen sauren Umesterungskatalysator zugibt und dann die 2-(Perfluoralkyl)-äthylene durch fraktionierte Destillation selektiv entfernt.

Bei dieser erfindungsgemäßen Arbeitsweise verbleiben die $R_f$-äthanole im Rückstand, während die $R_f$-äthylene zusammen mit dem als Schleppmittel zugesetzten Alkanol oder Äthylenglykol-monoalkyläther als Destillat erhalten werden, aus dem sie durch Ausfällen mit Wasser und durch Schichtentrennung isoliert werden können.

Die Wahl des als Schleppmittel für die $R_f$-äthylene eingesetzten Alkanols oder Äthylenglykol-monoalkyläthers richtet sich nach dem Hauptbestandteil hinsichtlich der Kettenlänge des $R_f$-Rests, der in den Komponenten des Gemisches vorkommt. Überwiegen hier $R_f$-Reste mit Kettenlängen von etwa 6 bis etwa 10 C-Atomen, so werden bevorzugt Methanol, Äthanol oder Isopropanol als Schleppmittel zugesetzt. Überwiegen im Gemisch jedoch $R_f$-Reste mit Kettenlängen von mehr als 10 C-Atomen, so gibt man zweckmäßigerweise Propanol oder Alkanole mit 4 bis 8 C-Atomen oder die Monomethyl-, Monoäthyl-, Monopropyl- oder Monobutyläther des Äthylenglykols zu. Zur besseren Abstimmung des Schleppverhaltens können gegebenenfalls auch Gemische der genannten Alkanole und/oder Glykoläther verwendet werden.

Die Menge des als Schleppmittel zugesetzten Alkanols oder Äthylenglykol-monoalkyläthers kann in ziemlich weiten Grenzen schwanken, sie beträgt etwa von 1 : 10 bis 10 : 1 Volumenteile, bezogen auf das Volumen der zu trennenden Ausgangsgemische. Bevorzugt wird ein Volumenverhältnis von Schleppmittel zu eingesetztem Ausgangsgemisch von 1 : 5 bis 5 : 1. Größere Mengen an Schleppmittel (als 10 : 1) sind möglich, bringen aber keinen weiteren Vorteil.

Sofern die zu trennenden Ausgangsgemische neben $R_f$-äthanolen und $R_f$-äthylenen auch noch $R_f$-äthylester enthalten, ist neben dem Schleppmittel ein saurer Umesterungskatalysator zuzugeben. Ein solcher saurer Umesterungskatalysator kann beispielsweise sein konzentrierte Schwefelsäure, Phosphorsäure, eine Alkyl-, Alkylaryl- oder Arylsulfonsäure oder auch ein saure Gruppen abgebender Ionenaustauscher. Die erforderliche Menge an diesem sauren Umesterungskatalysator beträgt etwa 1 bis 50 Gew.-%, bezogen auf die im Ausgangsgemisch anwesende Menge an $R_f$-äthylester.

Zur Durchführung des erfindungsgemäßen Verfahrens werden das zu trennende Ausgangsgemisch und das Schleppmittel in einen Destillationskolben gegeben, der mit einem Rührer und einer gut wirksamen Destillationskolonne, welche die erforderliche Trennleistung erbringt, ausgerüstet ist. Zur Verkleinerung des Gesamtvolumens kann man am Anfang auch nur einen Teil des Schleppmittels vorlegen und den restlichen Anteil während der Destillation gegebenenfalls portionsweise zugeben, während der saure Umesterungskatalysator zweckmäßigerweise bei Destillationsbeginn hinzugefügt wird. Unter Rühren destilliert man dann das Gemisch von Schleppmittel und $R_f$-äthylenen ab, wobei gleichzeitig gegebenenfalls vorhandener $R_f$-äthylester umgeestert wird.

Besteht das zu trennende Ausgangsgemisch nur aus $R_f$-äthanolen und $R_f$-äthylenen, so kann man es auch zusammen mit dem Schleppmittel kontinuierlich einer Destillationskolonne zuführen, an deren Kopf dann das Gemisch von $R_f$-äthylenen und Schleppmittel und von deren Sumpf die $R_f$-äthanole abgezogen werden.

Die im Rückstand befindlichen $R_f$-äthanole werden von geringen darin verbliebenen Restmengen des Schleppmittels durch Destillation befreit. Der gegebenenfalls in diesem Rückstand befindliche Umesterungskatalysator kann zweckmäßigerweise entweder durch Auswaschen mit Wasser (Säuren) oder durch Abfiltrieren (saure Ionenaustauscher) befreit werden.

Da die Anlagerung des Äthylens an Perfluoralkyljodide ebenfalls eine Telomerisationsreaktion darstellt, entstehen dabei auch Produkte der Formel $R_f(CH_2CH_2)_nJ$, in denen n Werte von 2 und größer als 2 besitzt. Diese werden vor der Weiterverarbeitung jedoch üblicherweise destillativ abgetrennt, wobei nur geringe Mengen (bis höchstens 3 Gew.-%) an Verbindungen, die 2 angelagerte Äthyleneinheiten enthalten, in den $R_f$-äthyljodiden verbleiben. Diese sind dann auch in den daraus entstandenen Komponenten der Ausgangsgemische enthalten. Diese Verbindungen stören die Trennung jedoch nicht.

Die nach dem erfindungsgemäßen Verfahren aus den Gemischen erhaltenen reinen $R_f$-äthanole wie auch die $R_f$-äthylene sind wertvolle Zwischenprodukte für die Herstellung von hydrophobierenden, oleophobierenden und/oder schmutzabweisenden Textilausrüstungsmitteln sowie für die Herstellung von Feuerlöschmittelzusätzen. Beispielsweise können aus den $R_f$-äthanolen deren Ester mit ungesättigten Carbonsäuren, insbesondere mit Acryl- oder Methacrylsäure, hergestellt werden, aus denen durch Polymerisation wichtige Textilausrüstungsmittel erhältlich sind.

Die folgenden Beispiele sollen das erfindungsgemäße Verfahren näher erläutern:

Beispiel 1

Zur Umsetzung mit Kaliumacetat in Äthanol bei 200 °C wird ein Gemisch von Perfluoralkyläthyljodiden folgender Kettenverteilung verwendet:

| | |
|---|---|
| $C_6F_{13}CH_2CH_2J$ | 48,3 Gew.-% |
| $C_8H_{17}CH_2CH_2J$ | 33,4 Gew.-% |
| $C_{10}F_{21}CH_2CH_2J$ | 13,0 Gew.-% |
| $C_{12}F_{25}CH_2CH_2J$ | 5,3 Gew.-% |

Das resultierende Gemisch hat folgende Zusammensetzung:

| | |
|---|---|
| $R_fCH=CH_2$ | 19,5 Gew.-% |
| $R_fCH_2CH_2OCOCH_3$ | 62,0 Gew.-% |
| $R_fCH_2CH_2OH$ | 18,5 Gew.-% |

Die Kettenverteilung der $R_f$-Reste ist die gleiche wie oben für die $R_f$-Jodide angegeben. 500 g (310 ml) dieses Gemisches werden zusammen mit 2 l Methanol und 10 g konzentrierter Schwefelsäure in einen 4 l-Glaskolben gegeben, der mit Rührer, Thermometer und einer 25 cm-Spiegelglaskolonne mit Destillationsaufsatz ausgestattet ist. Der Kolbeninhalt wird unter Rühren zum Sieden erhitzt und eine Stunde am Rückfluß gekocht. Anschließend werden das entstandene Methylacetat (Kp. 57 °C) und das die $R_f$-äthylene enthaltende Methanol abdestilliert (Sumpftemperatur maximal 70 °C).

Durch Zugabe von Wasser zum Destillat scheiden sich 93 g reine $R_f$-äthylene als untere Phase ab.

Der Kolbenrückstand von 407 g wird durch dreimaliges Ausrühren mit je 2 l Wasser bei 70 °C gewaschen, so daß er frei von Methanol und Schwefelsäure ist, und dann ohne Kolonne im Vakuum destilliert.

Man erhält:

|  | Sumpftemp. °C | Kp °C | Druck mbar | Menge g |
|---|---|---|---|---|
| Vorlauf | bis 80 | bis 70 | 10 | 4 (Wasser) |
| Hauptfraktion | 80 - 170 | 70 - 135 | 10 | 395 |
| Rückstand |  |  |  | 8 |

Die gaschromatographische Analyse der Hauptfraktion ergibt:

| | |
|---|---|
| $R_fCH_2CH_2OH$ | 98,7 Gew.-% |
| $R_fCH=CH_2$ | 0,8 Gew.-% |
| $R_fCH_2CH_2OCOCH_3$ | 0,5 Gew.-% |

(Kettenverteilung der $R_f$-Reste wie oben)

Beispiel 2

Es wird ein Gemisch von $R_f$-äthylenen, -äthanolen und -äthylestern eingesetzt, mit 2 l Methanol versetzt und aufgearbeitet, wie es in Beispiel 1 beschrieben ist, jedoch werden statt 10 g konzentrierter $H_2SO_4$ hier 40 g eines sauren Ionenaustauschers als Umesterungskatalysator verwendet (Bezeichnung Amberlyst® 15, Hersteller: Rohm and Haas, Philadelphia, USA).

Das Methanol wird zusammen mit Methylacetat und den $R_f$-äthylenen vollständig abdestilliert (Sumpftemperatur maximal 120 °C).

Die $R_f$-äthylene werden mit Wasser aus dem Destillat ausgefällt und sind frei von $R_f$-äthanolen.

Der Ionenaustauscher wird vom Kolbenrückstand abfiltriert. Dieser Rückstand (410 g) zeigt im Gaschromatogramm folgende Zusammensetzung:

| | |
|---|---|
| $R_f CH_2 CH_2 OH$ | 98,6 Gew.-% |
| $R_f CH=CH_2$ | 0,8 Gew.-% |
| $R_f CH_2 CH_2 OCOCH_3$ | 0,6 Gew.-% |

(Kettenverteilung der $R_f$-Reste wie im Ausgangsprodukt).


**Beispiel 3**

Zur Umsetzung mit Kaliumacetat in Äthanol wird ein Gemisch von Perfluoralkyläthyljodiden folgender Kettenverteilung verwendet:

| | |
|---|---|
| $C_6 F_{13} CH_2 CH_2 J$ | 41,4 Gew.-% |
| $C_8 F_{17} CH_2 CH_2 J$ | 29,9 Gew.-% |
| $C_{10} F_{21} CH_2 CH_2 J$ | 17,9 Gew.-% |
| $C_{12} F_{25} CH_2 CH_2 J$ | 8,2 Gew.-% |
| $C_{14} F_{29} CH_2 CH_2 J$ | 2,4 Gew.-% |
| $C_{16} F_{33} CH_2 CH_2 J$ | 0,2 Gew.-% |

Das resultierende Gemisch hat folgende Zusammensetzung:

| | |
|---|---|
| $R_f CH=CH_2$ | ·19,0 Gew.-% |
| $R_f CH_2 CH_2 OCOCH_3$ | 62,0 Gew.-% |
| $R_f CH_2 CH_2 OH$ | 19,0 Gew.-% |

(Kettenverteilung der $R_f$-Reste wie oben).


500 g (310 ml) dieses Gemisches werden zusammen mit 2 l n-Butanol und 10 g konzentrierter Schwefelsäure in die gleiche Apparatur gegeben, wie sie im Beispiel 1 verwendet wurde, die jedoch diesmal mit einer 50 cm-Spiegelglaskolonne und Rückflußkühler ausgerüstet ist.

Der Kolbeninhalt wird unter Rühren am Rückfluß gekocht, wobei sich im Phasenabscheider die $R_f$-äthylene als untere und n-Butanol als obere Schicht abtrennen. Nach zweistündigem Kochen ist die Phasenbildung beendet.

In weiteren zwei Stunden werden 90 % des Butanols abdestilliert (Sumpftemperatur maximal 120 °C).

Die gaschromatographische Analyse ergibt, daß das Butanoldestillat aus reinem Butanol und die untere Phase aus 17 Gew.-% Butanol, 4,7 Gew.-% Butylacetat und 78,3 Gew.-% $R_f$-äthylenen besteht.

Der Kolbenrückstand von 410 g wird durch dreimaliges Ausrühren mit je einem Liter Wasser frei von Säure gewaschen und ohne Kolonne im Vakuum destilliert.

Man erhält:

|  | Kp °C | Druck mbar | Menge g |
|---|---|---|---|
| Vorlauf | bis 70 | 10 | 8 (Butanol/Wasser) |
| Hauptfraktion | 70 - 165 | 10 | 392 |
| Rückstand |  |  | 10 |

Die gaschromatographische Analyse der Hauptfraktion ergibt:

| | |
|---|---|
| $R_fCH_2CH_2OH$ | 99,5 Gew.-% |
| $R_fCH=CH_2$ | 0,5 Gew.-% |

(Kettenverteilung der $R_f$-Reste wie oben).

Beispiel 4

Man verwendet ein Gemisch von 450 g $R_f$-äthanolen und 50 g $R_f$-äthylenen ($R_f$-Kettenverteilung wie in Beispiel 1; zusammen 300 ml) und arbeitet in der gleichen Apparatur wie im Beispiel 1, jedoch mit einer 50 cm-Kolonne.

Nach Zugabe von 1 l Methanol wird unter Rühren am Rückfluß gekocht und anschließend das Methanol bis zu einer Sumpftemperatur von 120 °C vollständig abdestilliert. Durch Wasserzugabe in das Methanoldestillat scheiden sich 48 g $R_f$-äthylene ab.

Der Kolbenrückstand von 452 g besteht aus

$$99,2 \text{ Gew.-\%} \qquad R_fCH_2CH_2OH \quad \text{und}$$

$$0,8 \text{ Gew.-\%} \qquad R_fCH=CH_2$$

Beispiel 5

Eingesetzt wird das Produkt aus der Umsetzung von Perfluoralkyljodiden ($R_f$-Kettenverteilung wie im Beispiel 1) mit Dimethylformamid und Wasser. Dieses Gemisch besteht aus:

| | |
|---|---|
| $R_fCH_2CH_2OH$ | 70 Gew.-% |
| $R_fCH_2CH_2OCHO$ | 20 Gew.-% |
| $R_fCH=CH_2$ | 10 Gew.-% |

($R_f$-Kettenverteilung wie in Beispiel 1).

250 g (155 ml) dieses Gemisches werden mit 0,5 l Methanol und 2,5 ml konzentrierter Schwefelsäure unter Rühren eine Stunde am Rückfluß gekocht. Anschließend werden das entstandene Methylformiat und das die $R_f$-äthylene enthaltende Methanol bis zu einer Sumpftemperatur von 70 °C abdestilliert.

Durch Wasserzugabe zum Destillat scheiden sich 24 g reine $R_f$-äthylene als untere Phase ab.

Der Kolbenrückstand wird durch dreimaliges Auswaschen mit je 250 ml Wasser frei von Methanol und Säure gewaschen.

Gemäß gaschromatographischer Analyse besteht er aus:

99,3 Gew.-%          $R_fCH_2CH_2OH$

0,4 Gew.-%          $R_fCH=CH_2$

0,3 Gew.-%          $R_fCH_2CH_2OCHO$

($R_f$-Kettenverteilung wie oben).

Beispiel 6

Eingesetzt wird das Produkt der Umsetzung von Perfluoralkyläthyljodiden (Kettenverteilung wie im Beispiel 1) mit p-Toluolsulfonsäure und Wasser. Dieses Gemisch besteht aus:

$R_fCH_2CH_2OH$          92,0 Gew.-%

$R_fCH=CH_2$          8,0 Gew.-%

($R_f$-Kettenverteilung wie Beispiel 1).

360 g (225 ml) dieses Gemisches werden mit 0,3 l Methanol am Rückfluß gekocht. Dann wird das Methanol abdestilliert, wobei weitere 0,7 l Methanol über einen Tropftrichter hinzugegeben werden.

Bei einer Sumpftemperatur von 120 °C wird die Destillation beendet.

Durch Wasserzugabe zum Methanoldestillat scheiden sich 28 g reine $R_f$-äthylene als untere Phase ab. Der Kolbenrückstand von 332 g besteht aus:

99,2 Gew.-%          $R_fCH_2CH_2OH$

0,8 Gew.-%          $R_fCH=CH_2$

($R_f$-Kettenverteilung wie oben).

Beispiel 7

Zur Umsetzung mit Dimethylformamid und Wasser wird ein Gemisch von Perfluoralkyläthyljodiden folgender Kettenverteilung verwendet.

| | |
|---|---|
| $C_6F_{13}CH_2CH_2J$ | 42,0 Gew.-% |
| $C_8F_{17}CH_2CH_2J$ | 28,2 Gew.-% |
| $C_{10}F_{21}CH_2CH_2J$ | 14,9 Gew.-% |
| $C_{12}F_{25}CH_2CH_2J$ | 8,7 Gew.-% |
| $C_{14}F_{29}CH_2CH_2J$ | 3,5 Gew.-% |
| $C_{16}F_{33}CH_2CH_2J$ | 1,5 Gew.-% |
| höhere Homologe | |
| (bis $C_{24}F_{49}$) | 1,2 Gew.-% |

Das aus dieser Umsetzung resultierende Gemisch hat folgende Zusammensetzung:

| | |
|---|---|
| $R_fCH_2CH_2OH$ | 77,4 Gew.-% |
| $R_fCH=CH_2$ | 10,7 Gew.-% |
| $R_fCH_2CH_2OCHO$ | 11,9 Gew.-% |

1000 g (625 ml) dieses Gemisches werden zusammen mit 400 g (416 ml) Äthylenglykol-monomethyläther (Kp 124,5 °C) und 5 g konzentrierter Schwefelsäure in einen 2 1-Kolben gegeben, der mit Rührer, Thermometer und einer 25 cm-Spiegelglaskolonne mit Phasenabscheider ausgestattet ist. Der Kolbeninhalt wird unter Rühren eine Stunde am Rückfluß gekocht. Dabei scheiden sich im Phasenabscheider 100 g reine $R_f$-äthylene als untere Phase ab. Dann wird der Glykoläther bis zu einer Sumpftemperatur von 130 °C vollständig abdestilliert.

Das Glykolätherdestillat enthält nach gaschromatographischer Analyse etwas $C_6F_{13}CH_2CH_2OH$, das durch Ausfällen mit Wasser als untere Phase gewonnen wird (40 g).

Der Kolbenrückstand wird durch dreimaliges Ausrühren mit je 2 1 Wasser säurefrei gewaschen und anschließend ohne Kolonne bei vermindertem Druck destilliert.

Man erhält:

| | Kp °C | Druck mbar | Menge g |
|---|---|---|---|
| Vorlauf | bis 90 | 10 | 30 |
| Hauptfraktion | 90 - 145 | 10 | 770 |
| Rückstand | | | 25 |

Nach der gaschromatographischen Analyse besteht die Hauptfraktion aus reinem $R_f CH_2 CH_2 OH$.

Der $R_f$-äthylester ist vollkommen in $R_f$-äthanol übergegangen, der $R_f$-äthylen-Gehalt liegt unter der Nachweisgrenze. Die $R_f$-Kettenverteilung in $R_f$-äthanol, in der Hauptfraktion und im Rückstand entspricht (unter Berücksichtigung des mit dem Glykolätherdestillat übergegangenen Anteils) derjenigen im Ausgangsprodukt, wobei sich die Homologen mit $R_f$-Ketten von 16 und mehr C-Atomen vorwiegend im Rückstand befinden.

## Beispiel 8

Eingesetzt wird das Umsetzungsprodukt von Perfluoralkyläthyljodiden ($R_f$-Kettenverteilung wie im Beispiel 1) mit Kaliumacetat in Äthanol bei 200 °C. Dieses Gemisch besteht aus:

| | |
|---|---|
| $R_f CH=CH_2$ | 19,0 Gew.-% |
| $R_f CH_2 CH_2 OCOCH_3$ | 62,0 Gew.-% |
| $R_f CH_2 CH_2 OH$ | 19,0 Gew.-% |

500 g (310 ml) dieses Gemisches werden zusammen mit 1 l Äthylenglykol-monoäthyläther (Kp 135 °C) und 5 g konzentrierter Schwefelsäure unter Rühren 3 Stunden am Rückfluß gekocht, wobei die $R_f$-äthylene ausgekreist werden. Danach werden 800 ml des Äthylglykols bei Normaldruck, die restlichen 200 ml bei 12 mbar abdestilliert (Sumpftemperatur maximal 132 °C).

Aus dem Glykolätherdestillat werden durch Wasserzugabe 28 g $C_6 F_{13} CH_2 CH_2 OH$ als untere Phase abgetrennt. Der Kolbenrückstand wird durch zweimaliges Ausrühren mit Wasser bei 70 °C frei von Glykoläther und Säure gewaschen und dann ohne Kolonne destilliert.

Man erhält:

|  | Kp °C | Druck mbar | Menge g |
|---|---|---|---|
| Vorlauf | bis 70 | 10 | 4,8 |
| Hauptfraktion | 70 - 121 | 10 | 347,0 |
| Rückstand |  |  | 20,5 |

Nach der gaschromatographischen Analyse besteht die Hauptfraktion aus

$R_f CH_2 CH_2 OH$     98,9 Gew.-%

$R_f CH_2 CH_2 OCOCH_3$     0,6 Gew.-%

Der $R_f$-äthylen-Gehalt liegt unter der Nachweisgrenze

($R_f$-Kettenverteilung wie oben unter Berücksichtigung des mit dem Glykolätherdestillat übergegangenen Anteils).

Die gaschromatographischen Analysen wurden unter folgenden Bedingungen durchgeführt:
Länge der Säule 3,65 m;
Durchmesser 0,32 cm; gefüllt mit Chromosorb® vom Typ WAW DMCS; stationäre Phase 10 Gew.-% Vinylmethylsilicon (UCCW 982); Trägergas: Stickstoff mit einer Geschwindigkeit von 20 ml/min.

Patentansprüche:

1. Verfahren zur Gewinnung von 2-(Perfluoralkyl)-äthanolen der Formel $R_f CH_2 CH_2 OH$ aus deren Gemischen mit 2-(Perfluoralkyl)-äthylenen der Formel $R_f CH=CH_2$ und gegebenenfalls 2-(Perfluoralkyl)-äthylcarbonsäureestern der Formel $R_f CH_2 CH_2 OCOR$, wobei $R_f$ in diesen Formeln ein Gemisch aus Perfluoralkylresten mit 4 bis 24 C-Atomen, die geradkettig oder endständig methylverzweigt sind, und wobei R Wasserstoff, einen aliphatischen oder aromatischen Rest bedeutet, dadurch gekennzeichnet, daß man den Gemischen ein Alkanol mit 1 bis 8 C-Atomen oder einen Monoalkyläther des Äthylenglykols, dessen Alkylrest 1 bis 4 C-Atome besitzt oder deren Gemische als Schleppmittel und bei Anwesenheit von 2-(Perfluoralkyl)-äthylcarbonsäureestern ferner einen sauren Umesterungskatalysator zusetzt und dann die 2-(Perfluoralkyl)-äthylene durch fraktionierte Destillation selektiv entfernt.

2. Verfahren nach Anspruch 1, dadurch gekennzeichnet, daß man das Schleppmittel dem zu trennenden Gemisch in einem Volumenverhältnis von 1 : 10 bis 10 : 1 zusetzt.

Europäisches
Patentamt

**EUROPÄISCHER RECHERCHENBERICHT**

EP 79 102 589.3

| EINSCHLÄGIGE DOKUMENTE | | | KLASSIFIKATION DER ANMELDUNG (Int.Cl.) |
|---|---|---|---|
| Kategorie | Kennzeichnung des Dokuments mit Angabe, soweit erforderlich, der maßgeblichen Teile | betrifft Anspruch | |
| A,P | DE - A1 - 2 811 462 (MITSUI PETROCHEMI- CAL INDUSTRIES) <br> * Anspruch 1; Seite 7, Zeilen 10 bis 20; Seite 8, Zeilen 7 bis 16 * <br> -- | 1 | C 07 C 29/82 <br> C 07 C 31/40 |
| A,D | DE - A - 2 318 677 (HOECHST) <br> * Anspruch 1 * <br> ---- | 1 | |

**RECHERCHIERTE SACHGEBIETE (Int. Cl.)**

C 07 C 29/82
C 07 C 31/38
C 07 C 31/40

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie. Übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 13-11-1979 | KNAACK |

EPA form 1503.1 06.78